# EUROPEAN PATENT APPLICATION

(11) **EP 4 806 438 A1**
(43) Date of publication of application: **16.09.2026**
(21) Application number: 25162959.8
(22) Date of filing: 11.03.2025
(51) Int. Cl.: A61K 31/506, A61K 31/517, A61K 31/4155, A61K 31/4545, A61K 45/06, A61P 35/00, A61P 35/04

(54) **PHARMACEUTICAL COMBINATION PRODUCT COMPRISING AN INHIBITOR OF A TYROSINE KINASE AND AN AUTOPHAGY INDUCER**

(71) Applicant: Universität Duisburg-Essen (Körperschaft des Öffentlichen Rechts), 45141 Essen (DE)
(72) Inventor: Peña-Llopis, Samuel, 45147 Essen (DE); Vega-Rubin-de-Celis, Silvia, 45147 Essen (DE)
(74) Representative: Michalski Hüttermann & Partner mbB

(57) **Abstract**

The present invention relates to a pharmaceutical combination product comprising: a) an inhibitor of a tyrosine kinase selected from human epidermal growth factor receptor (EGFR) family members epidermal growth factor receptor (EGFR) and erb-b2 receptor tyrosine kinase (HER2), or anaplastic lymphoma kinase (ALK); and b) an autophagy inducer as defined herein, preferably a Beclin1-dependent autophagy inducer.

## Description

The present invention relates to the field of treatment of cancer.

Lung and breast cancer are the most commonly diagnosed cancers worldwide. About 20% of non-small cell lung cancer (NSCLC), which represents 85% of all lung cancers, have mutations in *HER1 (ERBBl, EGFR),* and 20% of breast cancers have amplifications in *HER2 (ERBB2),* which correlates with more aggressive disease. HER2 amplifications are also found in about 10-30% of gastroesophageal as well as 15% of bladder cancers. Both HER1 and HER2 belong to the receptor tyrosine kinase (RTK) of the epidermal growth factor receptor (EGFR) family. The human epidermal growth factor receptor (EGFR) family contains four tyrosine kinases that belong to the ErbB lineage of proteins (ErbB1-4). In humans, the family includes Her1 (EGFR, ErbB1), Her2 (ErbB2), Her3 (ErbB3), and Her4 (ErbB4). These tyrosine kinase receptors trigger intracellular signals at the origin of essential cellular functions, including differentiation, proliferation, survival, and migration, and they regulate cell growth and survival through different signal transduction pathways, including PI3K/Akt and Ras/MAPK. Excessive ErbB signaling is associated with the development of a wide variety of types of solid tumors. Of the ERBB family members, HER2 and EGFR are frequently overexpressed or contain activating mutations in various cancers, including breast cancer, and are currently targets of many drugs, for example, tyrosine kinase inhibitors (TKIs).

Other RTKs such as ALK (anaplastic lymphoma kinase) are also involved in multiple forms of lung cancer, including malignant pleural mesothelioma. ALK positive lung cancer represents around 15% of the total cases worldwide. Targeted therapies with HER2 / EGFR / ALK inhibitors are currently applied in clinical practice, including humanized monoclonal antibodies against the HER2 extracellular domain such as trastuzumab and pertuzumab, and small molecule ATP-competitive tyrosine kinase inhibitors such as lapatinib, erlotinib, and crizotinib. However, such therapies produce very adverse side effects, and most tumors develop resistance over time. Furthermore, in the metastatic setting, the benefits of these therapies are limited.

*BECN1* is a haploinsufficient tumor suppressor, and although is not frequently mutated in cancer, *becn1+*/*-* mice show an increased incidence of spontaneous tumors. Beclin 1, also denoted BECN1, the protein encoded by the *BECN1* gene, is essential for autophagy. Autophagy is an intracellular degradation process in which the engulfment of cytoplasmic and organelle components by double-membraned vesicles (autophagosomes) leads to their degradation in the lysosome. Autophagy is an evolutionary conserved pathway that is implicated in many physiological (such as starvation, removal of damaged organelles, exercise) as well as pathological conditions, including protection against diseases, such as cancer. BECN1 is part of the Class **III** PI3K complex and is essential at the initial stages of the autophagosome formation. Autophagy de-regulation has been shown in cancer, but the specific function of autophagy in each cancer type is still obscure and whether autophagy plays a "protecting" or a "promoting" role in cancer remains to be determined.

Interplay between receptor tyrosine kinases and autophagy has been reported. In a review paper, Elshazly, A.M. et al., Cancers 2024, 16, 2989, describe that targeting or modulating autophagy can potentially enhance the tumor cell response to tyrosine kinase inhibitors, such as treatment with the early-stage autophagy inhibitor, VPS34-IN1 in combination with lapatinib.

J. Montero-Vergara et al., Cell Death and Disease (2024) 15:14, reported GRB2 being an adaptor protein of HER2 and several other tyrosine kinases, which was identified as a novel BECN1 (Beclin 1) interacting partner. In a short overview of this publication, S. Vega-Rubin-de-Celis, Autophagy Reports 2024, Vol. 3, No. 1, 2325265, highlighted the importance of the interaction between GRB2 and BECN1 in cancer. Vega-Rubin-de-Celis, S. et al., Increased autophagy blocks HER2-mediated breast tumorigenesis, Proc. Natl. Acad. Sci. USA 115, 4176-4181, (2018) demonstrated that HER2 binds to BECN1 and inhibits autophagy, while Tat-Beclin 1 autophagy-inducing peptide was able to dissociate HER2/Beclin 1 binding in tumor xenografts and stop tumor growth. It was reported that treatment of xenografts overexpressing HER2 with an autophagy-inducing peptide prevents tumor growth. It was further shown that treatment with receptor tyrosine kinase inhibitor lapatinib induced autophagy.

Wei, Y. et al., EGFR-mediated Beclin 1 phosphorylation in autophagy suppression, tumor progression, and tumor chemoresistance. Cell 154, 1269-84, (2013) showed that treatment with receptor tyrosine kinase inhibitor erlotinib induced autophagy. J. Chen et al., Biochemical and Biophysical Research Communications 636 (2022) 125-131, title that a Beclin 1-targeting stapled peptide synergizes with erlotinib to potently inhibit proliferation of non-small-cell lung cancer cells.

US 2024/0190920 A1 discloses Beclin 1-targeting stapled peptide induced autophagy and also enhanced the endolysosomal degradation of cell surface oncogenic receptors EGFR and HER2 in HER2-positive cancer cells.

WO 2021/055562 A1 describes a method of enhancing the response to an anti-cancer therapy with modulators, which can be an autophagy modulator.

PCT/EP2024/075359 describes a combination of an inhibitor of non-receptor tyrosine kinase SRC and an autophagy inducer for the treatment of cancer and a pharmaceutical combination product comprising an inhibitor of non-receptor tyrosine kinase SRC and an autophagy inducer.

Despite the described treatment options for cancer, there is a need for finding further pharmaceutical products for the treatment of cancer.

It is thus an object of the present invention to provide a further combination treatment for the treatment of cancer.

This object is achieved by a pharmaceutical combination product according to claim 1, an autophagy inducer for use in the treatment of cancer according to claim 11, and a use of an autophagy inducer for the manufacture of a pharmaceutical combination product for the treatment of cancer according to claim 12. Advantageous embodiments are the subject of dependent claims. They may be combined freely unless the context clearly indicates otherwise.

It has surprisingly been found that a combination of an inhibitor of tyrosine kinases EGFR, HER2 or ALK and a specific inducer of autophagy is effective in the treatment of cancer. Particularly, it was found that such combination had a synergistic effect *in vitro* in several cell lines with deregulated EGFR, HER2 and ALK tyrosine kinases, such as in case of non-small cell lung cancer cell lines with mutations in EGFR (H1975, H820 and HCC-827) treated with the EGFR inhibitor erlotinib and the autophagy inducers SW076956 and SW063058. Also, HER2+ breast cancer cell lines HCC-1954, JIMT-1 and MDA-MB-453 were found to show synergistic effects by a combination of the HER2 inhibitor lapatinib and the autophagy inducer SW076956. Similarly, the lung adenocarcinoma cell lines with alterations in tyrosine kinase ALK with resistance (PF240-CS) or sensitivity (PF521) to ALK inhibitors were found to show certain synergism by the combination of the ALK inhibitors ceritinib and crizotinib with the autophagy inducer SW076956.

According to a first aspect is provided a pharmaceutical combination product comprising:
a) an inhibitor of a tyrosine kinase selected from
   - human epidermal growth factor receptor (EGFR) family members epidermal growth factor receptor (EGFR) and erb-b2 receptor tyrosine kinase (HER2), or
   - anaplastic lymphoma kinase (ALK); and
b) an autophagy inducer selected from the group of:
   - SW076956 (CAS No. 851717-83-4) also denoted N-(4-(5-(furan-2-yl)-1-(4-methylbenzoyl)-4,5-dihydro-1H-pyrazol-3-yl)phenyl)methanesulfonamide;
   - SW063058 (CAS No. 533876-30-1) also denoted 7-Bromo-5-(4-fluorophenyl)-1,3,4,5-tetrahydro-4-(2-iodobenzoyl)-2H-1,4-benzodiazepin-2-one;
   - SW076956-35 also denoted isopropyl (4-(1-(4-bromobenzoyl)-5-phenyl-4,5-dihydro-1H-pyrazol-3-yl)phenyl)carbamate;
   - SW076956-24 also denoted tert-butyl (4-(1-(4-bromobenzoyl)-5-phenyl-4,5-dihydro-1H-pyrazol-3-yl)phenyl)carbamate;
   - BRD1991 (CAS No. 2235468-02-5) also denoted 3,5-dichloro-N-((10-(1-hydroxypropan-2-yl)-8,12-dimethyl-11-oxo-7,8,9,10,11,12-hexahydro-5H-benzo[9,10][1]oxa[5]azacycloundecino[7,8-b]indol-7-yl)methyl)-N-methylbenzamide;
   - BRD5631 (CAS No. 2446154-91-0) also denoted N-[(3 S,6S,7S)-7-methoxy-3,6,9-trimethyl-10-oxo-3,4,5,6,7,8,9,10-octahydro-2H-1,4,9-benzoxadiazacyclododecin-12-yl]-[ 1,1'-biphenyl]-4-carboxamide;
   - Tat-Beclin-1;
   - Tat-Beclin-1 peptide;
   - Tat-Beclin-1-D11 peptide;
   - Tat-Beclin-1-L11 peptide;
   - Tat-vFLIPα2; and
   - Tat-vFLIPα4,
   or a pharmaceutically acceptable salt or hydrate or prodrug thereof.

As used herein, the term "inhibitor of a tyrosine kinase" refers to a compound that blocks the catalytic activity of the tyrosine kinase. For instance, it can compete with adenosine triphosphate (ATP), which is involved in adding phosphate groups, either as the substrate or as the phosphorylating entity. Alternatively, it can work in a different way by binding to a different site on the enzyme, called an allosteric site, and affecting its activity by causing a conformational change. The term "inhibitor of a tyrosine kinase" particularly refers to small-molecule kinase inhibitors, which usually are denoted "tyrosine kinase inhibitor" (TKI), and monoclonal antibodies. TKIs and monoclonal antibodies both can bind to a, intracellular or extracellular, domain of a tyrosine kinase and inhibit the further signal transduction. A primary action mechanism of most antibodies is a competitive antagonism on the tyrosine kinase.

As used herein, the term "autophagy inducer" refers to a compound that is able to promote autophagy flux by targeting regulators of the autophagy process and/or parts of the molecular autophagy machinery itself to specifically induce autophagy without affecting other processes, e.g. without inducing apoptosis. Therefore, in this context, the term "autophagy inducer" refers to compounds which (A) target the binding of BECN1 to Bcl-2 (B-cell lymphoma 2) without affecting apoptosis, such as SW076956, SW063058, SW076956-35, SW076956-24, BRD1991 and BRD5631, or (B) which target the binding of Beclin-1 to GAPR-1/GLIPR2 (Golgi-Associated Plant Pathogenesis-Related Protein 1/Glioma Pathogenesis-Related Protein 2) without affecting apoptosis, such as Tat-Beclin-1 peptide, Tat-Beclin-1-D11 or Tat-Beclin-1-L11; or (C) which target the binding of ATG3 (autophagy related 3) to LC3 (Microtubule-associated proteins 1A/1B light chain 3B), such as Tat-vFLIPα2 or Tat-vFLIPα4. In preferred embodiments, the autophagy inducer is selected from a compound that is able to promote autophagy flux by targeting the autophagy regulator BECN1 such as SW076956, SW063058, SW076956-35, SW076956-24, BRD1991 or BRD5631, and Tat-Beclin-1 peptide, Tat-Beclin-1-D11 or Tat-Beclin-1-L11. Thus, the autophagy inducer preferably is a Beclin1-dependent autophagy inducer.

The selection of the specific autophagy-inducing agents as defined above advantageously will minimize side effects by acting through other pathways, therefore maximizing the specificity of the treatments. Some FDA-approved drugs like lithium, metformin and statins usually have multiple, often non-specific effects, one of which can be used to trigger autophagy. However, given the multiple effects of such drugs, it is difficult to determine whether any beneficial effects seen in patients are the consequence of the activation of the autophagy machinery or of other mechanisms at play.

Therefore, when identifying compounds that specifically induce autophagy, it is crucial to prove that a compound's effects are indeed a direct consequence of the activation of the autophagy machinery. To illustrate such a direct and specific effect, compounds that specifically induce autophagy are unable to elicit their effects when the autophagy machinery is compromised, i.e. non-functional. In turn, non-specific signals that activate autophagy can also affect other pathways, such as the mammalian target of rapamycin complex 1 (mTORC1), the 5' AMP-activated protein kinase (AMPK) and protein kinase B (AKT) pathways. Therefore, it is important to distinguish between effects achieved because of the activation of autophagy or because of the modification of other pathways. For example, BH3 mimetic compounds disrupt the interaction between BECN1 and BECN1 inhibitors of the B-cell lymphoma 2 (Bcl-2) protein family and increase autophagy. However, such compounds are not autophagy inducers as understood in the context of the present disclosure as they also promote apoptosis by interrupting interactions between Bcl-2 family members and BH3 domains of molecules linked to apoptosis.

An autophagy inducer as used herein does not inhibit the catalytic activity of the tyrosine kinases HER1, HER2 or ALK. Pharmaceutically active ingredients that inhibit EGFR, HER2 or ALK and, coincidently, also induce autophagy are expressly excluded from the scope of the term "autophagy inducer" as used herein.

As used herein, the term "Tat-peptide" or "Tat-" refers to a cell penetrating peptide derived from the HIV-1 trans-activating transcriptional activator (Tat; UniProtKB: P35965) having the sequence X₁GRKKRRQRRRX₂X₃ of SEQ ID NO: 1 or the retro inverse sequence X₃X₂RRRQRRKKRGX₁ of SEQ ID NO: 2, where X₁ is either Y or absent, X₂ is either P or absent and X₃ is either Q or absent.

As used herein, the term "Tat-Beclin-1" refers to an N-terminal fusion of Tat-peptide, typically via a linker, preferably via a di-glycine linker, to full-length Beclin-1 protein of SEQ ID NO: 3 or a variant thereof having at least 80%, at least 85%, at least 90%, at least 95%, at least 96%, at least 97%, at least 98% or at least 99% sequence identity to SEQ ID NO: 3. For example, a variant of full-length Beclin-1 protein of SEQ ID NO: 3 may be SEQ ID NO: 4 or SEQ ID NO: 5 or SEQ ID NO: 6 or SEQ ID NO: 7 or SEQ ID NO: 8. In the context of the present invention, Tat-Beclin-1 and variants thereof maintain Beclin-1 function, namely Beclin-1 function relating to vesicle enucleation (PI3KC3-C1) as well as to autophagosome maturation and endocytic trafficking.

As used herein, the term "Tat-Beclin-1 peptide" refers to a peptide comprising: (1) Tat-peptide of SEQ ID NO: 1 or SEQ ID NO: 2; (2) a linker sequence, preferably a di-glycine linker; and (3) a Beclin-1 sequence comprising amino acid residues 267-284 of the autophagy protein Beclin-1 (TNVFNATFHIWHSGQFGT of SEQ ID NO: 9) or variants thereof having at least 80%, at least 85%, at least 90%, at least 95%, at least 96%, at least 97%, at least 98% or at least 99% sequence identity to SEQ ID NO: 9. A particularly useful variant of the Beclin-1 sequence of (3) above is the peptide variant TNVFNATFEIWHDGEFGT of SEQ ID NO: 10 having 83.33% sequence identity to SEQ ID NO: 9. The peptides X₁GRKKRRQRRRX₂X₃GGTNVFNATFX₄IWHX₅GX₆FGT of SEQ ID NO: 11, where X₁ is either Y or absent, X₂ is either P or absent, X₃ is either Q or absent, X₄ is either H or E, X₅ is either D or S, and X₆ is either E or Q, or X₁X₂RRRQRRKKRGX₃GGTNVFNATFX₄IWHX₅GX₆FGT of SEQ ID NO: 12, where X₁ is either Q or absent, X₂ is either P or absent, X₃ is either Y or absent, X₄ is either H or E, X₅ is either D or S, and X₆ is either E or Q, are express examples of Tat-Beclin-1 peptides encompassed by the above term.

Tat-Beclin-1-peptides are cell-penetrating peptides demonstrated to induce autophagy through interaction with the autophagy suppressor GAPR-1/GLIPR2 both *in vitro* and *in vivo.* Upon peptide binding, Beclin-1 bound to GARP-1 is released from the Golgi, resulting in Beclin-1 mediated autophagosome formation and autophagy induction. Illustrative of its specificity, i.e. as an autophagy inducer as understood herein, it is noteworthy that Tat-Beclin-1 peptides cannot affect autophagosome formation and autophagy induction in situations where sufficient amounts of Beclin 1 are lacking.

As used herein, the term "Tat-Beclin-1-D11" (NBP2-49888) refers to RRRQRRKKRGYGGDHWIHFTANWV of SEQ ID NO: 13, a shorter, re-engineered variant of the Tat-Beclin-1 peptide, only including 11 amino acids of Beclin-1, which demonstrates enhanced autophagy induction compared to the Tat-Beclin 1 peptide.

As used herein, the term "Tat-Beclin-1-L11" (NBP2-49886) refers to YGRKKRRQRRRGGVWNATFHIWHD of SEQ ID NO: 14, a shorter, re-engineered variant of the Tat-Beclin-1 peptide, only including 11 amino acids of Beclin-1, which demonstrates enhanced autophagy induction compared to the Tat-Beclin 1 peptide.

Both Tat-Beclin-1-D11 and Tat-Beclin-1-L11 peptides function by binding the negative regulator of autophagy GAPR-1/GLIPR2. Upon peptide binding, Beclin-1 bound to GARP-1 is released from the Golgi, resulting in Beclin-1 mediated autophagosome formation and autophagy induction.

As used herein, the term "Tat-vFLIPα2" refers to an N-terminal fusion of Tat-peptide to amino acids 20-29 (FVNLLFLVVE of SEQ ID NO: 15) of full length viral FLICE-like inhibitor protein (SEQ ID NO: 16 and 17, respectively).

As used herein, the term "Tat-vFLIPα4" in the context of this disclosure refers to an N-terminal fusion of Tat-peptide to amino acids 128-139 (NEMCYVWHLFTQ of SEQ ID NO: 18) of full length viral FLICE-like inhibitor protein (SEQ ID NO: 19 and 20, respectively).

Both Tat-vFLIPα2 and Tat-vFLIPα4 bind to the FLIP protein itself preventing its binding to autophagy related 3 (ATG3) and, in turn, allowing the binding of ATG3 to LC3 and thereby promoting the autophagosome formation and, as a consequence, the induction of autophagy.

A combination of an inhibitor of tyrosine kinases EGFR, HER2 or ALK with a specific inducer of autophagy as defined above provides several advantages. A combination of an inhibitor of tyrosine kinases EGFR, HER2 or ALK and such autophagy inducer will reduce the concentration required to reach maximal effects for each of them to be effective in the clinical setting. As such, the combination will minimize side effects. Further, patients that will benefit from a combination treatment with a tyrosine kinase inhibitor and an autophagy inducer are easy to identify, since HER1/HER2/ALK status is a standard procedure that is systematically and routinely determined for cancer patients whom are commonly having alterations in these receptor-tyrosine kinases. Thus, patient stratification and selection is guaranteed. A combination of an inhibitor of tyrosine kinases EGFR, HER2 or ALK with a specific inducer of autophagy moreover has the potential of also being effective in tumors that are resistant to conventional therapies, as was shown in ALK mutant mesothelioma cell lines.

Another advantage is that inhibitors of tyrosine kinases EGFR, HER2 or ALK, such as the following compounds targeting HER1, HER2 or ALK, are already in use in the clinic, so no further validation is required.

In embodiments, the inhibitor of the tyrosine kinase EGFR is selected from the group comprising or consisting of:
- erlotinib, also denoted 183321-74-6 or N-(3-ethynylphenyl)-6,7-bis(2methoxyethoxy)quinazolin-4-amine,
- gefitinib, also denoted 184475-35-2 or N-(3-chloro-4-fluorophenyl)-7-methoxy-6-(3-morpholin-4-ylpropoxy)quinazolin-4-amine,
- afatinib, also denoted BIBW2992 or (*E*)-*N*-[4-(3-chloro-4-fluoroanilino)-7-[(3*S*)-oxolan-3-yl]oxyquinazolin-6-yl]-4-(dimethylamino)but-2-enamide,
- dacomitinib, also denoted 1042385-75-0 or (E)-N-[4-(3-chloro-4-fluoroanilino)-7-methoxyquinazolin-6-yl]-4-piperidin-1-ylbut-2-enamide, preferably in the form of monohydrate,
- osimertinib, also denoted AZD-9291 or *N*-[2-[2-(dimethylamino)ethyl-methylamino]-4-methoxy-5-[[4-(1-methylindol-3-yl)pyrimidin-2-yl]amino]phenyl]prop-2-enamide, and
- canertinib, also denoted CI-1033 or N-[4-(3-chloro-4-fluoroanilino)-7-(3-morpholin-4-ylpropoxy)quinazolin-6-yl]prop-2-enamide,
- cetuximab,
- matuzumab,
- panitumumab,
- necitumumab,
or a pharmaceutically acceptable salt or hydrate or prodrug thereof.

Preferred tyrosine kinase EGFR inhibitors are selected from the group comprising or consisting of gefitinib, erlotinib, afatinib, dacomitinib, and osimertinib. The EGFR tyrosine kinase inhibitors gefitinib, erlotinib, afatinib, dacomitinib, and osimertinib are predominantly available in practice and are the first-line treatment of choice for patients with *EGFR* mutation-positive non-small cell lung cancer (NSCLC).

The terms "gefitinib", "erlotinib", "afatinib", "dacomitinib", "osimertinib" and "canertinib" as used herein encompass any enantiomers, tautomers, pharmaceutically acceptable salts, as well as solvates, hydrates and solvates of pharmaceutically acceptable salts thereof. In embodiments, a racemate may be used or a specific enantiomer of the compound. Erlotinib, sold under the brand name Arceva^{®} among others, is marketed by AstraZeneca and Teva. Gefitinib, sold under the brand name Iressa^{®}, is marketed by AstraZeneca and Teva. The EGFR inhibitor gefitinib is the first selective inhibitor of epidermal growth factor receptor's (EGFR) tyrosine kinase domain. Afatinib, sold under the brand names Giotrif^{®} or Gilotrif^{®}, is marketed by Boehringer-Ingelheim. Afatinib is used to treat metastatic NSCLC where tumours have activating EGFR mutations. Afatinib is also used to treat metastatic squamous NSCLC. Dacomitinib is sold under the brand name Vizimpro^{®}. Osimertinib is sold under the brand name Tagrisso^{®}. Osimertinib is a third-generation EGFR tyrosine kinase inhibitor, currently being the standard therapy for previously untreated EGFR-mutated non-small cell lung cancer patients. These EGFR tyrosine kinase inhibitors advantageously are available for oral administration.

Canertinib (CI-1033) is an experimental drug candidate for the treatment of cancer. Canertinib is a pan-erbB tyrosine kinase inhibitor with activity against EGFR and HER2. Canertinib may be used in the form of the dihydrochloride. Canertinib particularly has been shown to act as a potent and irreversible EGFR inhibitor.

In embodiments, the tyrosine kinase EGFR inhibitor is selected from the group comprising or consisting of cetuximab, matuzumab, panitumumab, and necitumumab. Cetuximab (CAS No. 205923-56-4) is a recombinant chimeric (mouse/human) monoclonal antibody targeting EGFR, which is sold, for example, under the trade name Erbitux^{®}. Cetuximab is used for treatment of colorectal cancer and squamous cell cancer of the head and neck. Matuzumab (CAS No. 339186-68-4), formerly EMD 72000, is a humanized monoclonal antibody targeting EGFR. Panitumumab (CAS No. 339177-26-3) is a human monoclonal antibody targeting EGFR, which is sold, for example, under the trade name Vectibix^{®}. Panitumumab was approved for treatment of refractory EGFR-expressing metastatic colorectal cancer. Necitumumab (INN) (CAS No. 906805-06-9), is a human recombinant monoclonal antibody, which is a EGFR antagonist. Necitumumab is available under the trade name Portrazza^{®}. Necitumumab is used for treatment of non-small cell lung cancer (NSCLC).

In embodiments, the inhibitor of the tyrosine kinase HER2 is selected from the group comprising or consisting of:
- lapatinib, also denoted GW572016 or N-[3-chloro-4-[(3-fluorophenyl)methoxy]phenyl]-6-[5-[(2-methylsulfonylethylamino)methyl]furan-2-yl]quinazolin-4-amine, preferably in the form of ditosylate,
- neratinib, also denoted HKI-272 or (*E*)-*N*-[4-[3-chloro-4-(pyridin-2-ylmethoxy)anilino]-3-cyano-7-ethoxyquinolin-6-yl]-4-(dimethylamino)but-2-enamide,
- tucatinib, also denoted ONT-380 or 6-N-(4,4-dimethyl-5*H*-1,3-oxazol-2-yl)-4-N-[3-methyl-4-([1,2,4]triazolo[1,5-a]pyridin-7-yloxy)phenyl]quinazoline-4,6-diamine,
- pyrotinib, also denoted SHR-1258 or (*E*)-*N*-[4-[3-chloro-4-(pyridin-2-ylmethoxy)anilino]-3-cyano-7-ethoxyquinolin-6-yl]-3-[(2*R*)-1-methylpyrrolidin-2-yl]prop-2-enamide,
- canertinib, also denoted CI-1033 or *N*-[4-(3-chloro-4-fluoroanilino)-7-(3-morpholin-4-ylpropoxy)quinazolin-6-yl]prop-2-enamide, preferably in the form of dihydrochloride, and
- afatinib, also denoted BIBW2992 or (*E*)-*N*-[4-(3-chloro-4-fluoroanilino)-7-[(3*S*)-oxolan-3-yl]oxyquinazolin-6-yl]-4-(dimethylamino)but-2-enamide,
- trastuzumab,
- pertuzumab,
- trastuzumab emtansine,
- trastuzumab deruxtecan,
or a pharmaceutically acceptable salt or hydrate or prodrug thereof.

In embodiments, the tyrosine kinase HER2 inhibitor is selected from the group comprising or consisting of lapatinib, neratinib, tucatinib, pyrotinib, canertinib, and afatinib. The terms "lapatinib", "neratinib", "tucatinib", "pyrotinib", "canertinib" and "afatinib" and as used herein encompass any enantiomers, tautomers, pharmaceutically acceptable salts, as well as solvates, hydrates and solvates of pharmaceutically acceptable salts thereof. In embodiments, a racemate may be used or a specific enantiomer of the compound.

Preferred tyrosine kinase HER2 inhibitors are selected from the group comprising lapatinib, neratinib and tucatinib. The tyrosine kinase inhibitors (TKIs) lapatinib, neratinib, and tucatinib are used for the treatment of HER2-positive breast cancer. Lapatinib is preferably used in the form of lapatinib ditosylate. Lapatinib ditosylate is sold under the brand names Tykerb^{®} and Tyverb^{®}. Lapatinib is used for the treatment of patients with advanced or metastatic breast cancer whose tumors overexpress HER2 (ErbB2). Neratinib is sold under the brand name Nerlynx^{®}. Tucatinib is sold under the brand name Tukysa^{®}.

Pyrotinib is a novel tyrosine kinase inhibitor that has shown efficacy in the treatment of HER2+ breast cancer. Canertinib (CI-1033) is an experimental drug candidate for the treatment of cancer. Canertinib is known to be a pan-erbB tyrosine kinase inhibitor with activity against EGFR and HER2. Canertinib may be used in the form of the dihydrochloride. Canertinib has been shown to have activity against HER2. Also afatinib is a pan-erbB tyrosine kinase inhibitor with activity against EGFR and HER2. Afatinib has a strong affinity for EGFR but also has shown to be a HER2 inhibitor.

In embodiments, the tyrosine kinase HER2 inhibitor is selected from the group comprising or consisting of trastuzumab, pertuzumab, trastuzumab emtansine, and trastuzumab deruxtecan. Trastuzumab (CAS No. 180288-69-1) is a recombinant humanized monoclonal antibody targeting HER2, which is sold, for example, under the trade name Herceptin^{®}. Trastuzumab is used for treatment of breast cancer and stomach cancer, specifically used for cancer that is HER2 receptor positive. Pertuzumab (CAS No. 380610-27-5) is a recombinant humanized monoclonal antibody targeting HER2. Pertuzumab is available under the trade name Perjeta^{®}. Pertuzumab is used for treatment of HER2-positive breast cancer. Trastuzumab emtansine (T-DM1, CAS No 1018448-65-1) is a conjugate of trastuzumab and emtansine (DM1), a microtubule inhibitor, which is sold, for example, under the trade name Kadcyla^{®}.

Trastuzumab emtansine was approved for treatment of HER2-positive metastatic breast cancer. Trastuzumab deruxtecan (T-DXd, DS-8201a, CAS No. 1826843-81-5 (ADC)), a conjugate of trastuzumab and deruxtecan, a topoisomerase I inhibitor payload. Trastuzumab deruxtecan is available under the trade name Enhertu^{®}. Trastuzumab deruxtecan is used for treatment of HER2-low metastatic breast cancer.

In embodiments, the inhibitor of the tyrosine kinase ALK is selected from the group comprising
- ceritinib, also denoted LDK-378 or 5-chloro-2-N-(5-methyl-4-piperidin-4-yl-2-propan-2-yloxyphenyl)-4-N-(2-propan-2-ylsulfonylphenyl)pyrimidine-2,4-diamine,
- crizotinib, also denoted PF-02341066 or 3-[(1R)-1-(2,6-dichloro-3-fluorophenyl)ethoxy]-5-(1-piperidin-4-ylpyrazol-4-yl)pyridin-2-amine,
- alectinib, also denoted 9-ethyl-6,6-dimethyl-8-(4-morpholinopiperidin-1-yl)-11-oxo-6,11-dihydro-5H-benzo[b]carbazole-3-carbonitrile,
- brigatinib, also denoted AP26113 or 4-amino-3-chloro-*N*-(4-(dimethylphosphinyl)phenyl)-N'-(2-methoxy-5-methylphenyl) pyrimidine-2-carboximidamide,
- lorlatinib, also denoted 3-[(2,6-difluorophenyl)methyl]-8-methyl-6-(4-morpholinopiperidin-1-yl)-2,4-dioxo-1,2,3,4-tetrahydropyrido[4,3-d]pyrimidine-7-carboxamide,
or a pharmaceutically acceptable salt or hydrate or prodrug thereof.

The terms "ceritinib", "crizotinib", "alectinib", "brigatinib" and "lorlatinib" and as used herein encompass any enantiomers, tautomers, pharmaceutically acceptable salts, as well as solvates, hydrates and solvates of pharmaceutically acceptable salts thereof. In embodiments, a racemate may be used or a specific enantiomer of the compound. Ceritinib, sold under the brand name Zykadia^{®}, is an ALK inhibitor primarily used for the treatment of ALK positive metastatic NSCLC. Crizotinib, sold under the brand name Xalkori^{®}, is also used for the treatment of ALK positive NSCLC. Alectinib (INN), available under the trade name Alecensa^{®}, is an ALK inhibitor used for the treatment of anaplastic lymphoma kinase (ALK)-positive advanced non-small cell lung cancer (NSCLC). Brigatinib (CAS No. 1197953-54-0), available under the trade name Alunbrig^{®}, is an ALK inhibitor used for the treatment of NSCLC. Lorlatinib (CAS No. 1454846-35-5), available under the trade names Lorbrena^{®} and Lorviqua^{®}, is an ALK inhibitor used for the treatment of metastatic NSCLC.

In preferred embodiments, the autophagy inducer is selected from the group comprising or consisting of SW076956, SW063058, SW076956-35, SW076956-24, BRD1991 and BRD5631. The terms "SW076956", "SW063058", "SW076956-35", "SW076956-24", "BRD1991" and "BRD5631" and as used herein encompass any enantiomers, tautomers, pharmaceutically acceptable salts, as well as solvates, hydrates and solvates of pharmaceutically acceptable salts thereof. In embodiments, a racemate may be used or a specific enantiomer of the compound. SW076956, SW063058, SW076956-35, SW076956-24, and BRD1991 are small molecules that selectively disrupt Beclin 1/Bcl-2 binding and induce autophagy without triggering apoptosis or other forms of cell death with minimal cytotoxicity. BRD5631 is an autophagy enhancer that operates through an mTOR-independent pathway. BRD5631 is known to stimulate formation of new autophagosomes. In preferred embodiments, the autophagy inducer is selected from the group comprising or consisting of SW076956 and SW063058. Particularly the autophagy inducers SW076956 and SW063058 were found to provide synergistic effects in combination with an inhibitor of tyrosine kinases EGFR, HER2 or ALK.

In a preferred embodiment, the inhibitor of the tyrosine kinase EGFR is erlotinib, and the autophagy inducer is SW076956. In another preferred embodiment, the inhibitor of the tyrosine kinase EGFR is erlotinib, and the autophagy inducer is SW063058. In non-small cell lung cancer cell lines with mutations in EGFR a combination of the EGFR inhibitor erlotinib and the autophagy inducer SW076956 provided synergistic effect *in vitro.*

In a preferred embodiment, the inhibitor of the tyrosine kinase HER2 is lapatinib, and the autophagy inducer is SW076956. In HER2+ breast cancer cell lines HCC-1954, JIMT-1 and MDA-MB-453 treated with the HER2 inhibitor lapatinib and the autophagy inducer SW076956 synergistic effect *in vitro* have been observed.

In a preferred embodiment, the inhibitor of the tyrosine kinase ALK is ceritinib and the autophagy inducer is SW076956. In another preferred embodiment, the inhibitor of the tyrosine kinase ALK is crizotinib and the autophagy inducer is SW076956. In lung adenocarcinoma cell lines with alterations in tyrosine kinase ALK with resistance (PF240-CS) or sensitivity (PF521) to ALK inhibitors certain synergism by the combination of the ALK inhibitors ceritinib and crizotinib with the autophagy inducer SW076956 were observed.

The term "pharmaceutical combination product" as used herein has its conventional meaning in that at least two different active ingredients being used in combination such that it refers either to a product in which a single composition comprises both active ingredients a) an inhibitor of the tyrosine kinase EGFR, HER2 or ALK and b) an autophagy inducer as defined above, or to a product, such as a kit, in which the two active ingredients a) an inhibitor of the tyrosine kinase EGFR, HER2 or ALK and b) an autophagy inducer as defined above are provided together but in separate compositions, possibly even for simultaneous or sequential administration via the same or a different route of administration.

In embodiments, the combination product may comprise the inhibitor of the tyrosine kinase EGFR, HER2 or ALK and the autophagy inducer as separate entities. Thus, the inhibitor of the tyrosine kinase may be administered independently of the autophagy inducer at the same time or separately within time intervals.

The combination product may be a kit comprising
- a first pharmaceutical composition or dosage form comprising the inhibitor of the tyrosine kinase EGFR, HER2 or ALK or a pharmaceutically acceptable salt, hydrate or prodrug thereof, and, optionally, pharmaceutically acceptable carriers, excipients and/or vehicles; and
- a second pharmaceutical composition or dosage form comprising the autophagy inducer or a pharmaceutically acceptable salt, hydrate or prodrug thereof, and, optionally, pharmaceutically acceptable carriers, excipients and/or vehicles.

The terms "first" and "second", as used herein, is solely intended to indicate that the compositions are two different compositions. Thus, these terms shall not be understood to refer to the order or sequence of administration.

The two separate entities of the pharmaceutical combination product, such as separate pharmaceutical compositions or dosage forms, may be formulated for the same or for distinct routes of administration. For example, the composition or dosage form comprising the inhibitor of the tyrosine kinase EGFR, HER2 or ALK may be formulated for intratumoral or intraperitoneal or systemic or intranasal or oral administration, while the autophagy inducer composition may be formulated for intratumoral or intraperitoneal or systemic or intranasal or oral administration. Further, the composition or dosage form comprising the inhibitor of the tyrosine kinase EGFR, HER2 or ALK may be formulated for oral administration and the composition or dosage form comprising the autophagy inducer may be formulated for intraperitoneal administration.

In embodiments, the composition or dosage form comprising the inhibitor of the tyrosine kinase EGFR, HER2 or ALK is formulated for oral or intravenous administration and the composition or dosage form comprising the autophagy inducer is formulated for oral or intravenous administration. Preferably, both compositions or dosage forms are formulated for oral administration, which has advantages in view of compliance. In other embodiments, one of the composition or dosage form comprising the inhibitor of the tyrosine kinase EGFR, HER2 or ALK or the composition or dosage form comprising the autophagy inducer may be formulated for oral administration, while the other may be formulated for intravenous administration.

In other embodiments, the combination product comprises the inhibitor of tyrosine kinase EGFR, HER2 or ALK and the autophagy inducer in a single composition. The single composition comprises the inhibitor of the tyrosine kinase EGFR, HER2 or ALK and the autophagy inducer may be formulated for joint oral or intraperitoneal or systemic administration.

The tyrosine kinase inhibitors or autophagy inducer may be usable in the form of a pharmaceutically acceptable salt or hydrate or prodrug thereof. The term "pharmaceutically acceptable salt" as used herein refers to salts which retain the biological effectiveness and properties of the free compound and which are not biologically or otherwise undesirable. A pharmaceutically acceptable salt can be conveniently prepared from pharmaceutically acceptable non-toxic bases, including inorganic and organic bases. Preferred salts derived from inorganic bases include ammonium, calcium, magnesium, potassium, and sodium salts. Salts derived from pharmaceutically acceptable organic, non-toxic bases include salts of primary, secondary, and tertiary amines and cyclic amines. Salts derived from pharmaceutically acceptable organic, non-toxic acids include tosylate salts or dihydrochloride salts. The term "hydrate" as used herein refers to an association or complex of water as a solvent molecule, such as a monohydrate of a compound. The term "prodrug" as used herein refers compound that is itself biologically inactive but is metabolized in the body to produce the active compound.

In embodiments, the pharmaceutical combination product comprises as an active ingredient a) an inhibitor of a tyrosine kinase selected from human epidermal growth factor receptor (EGFR) family members epidermal growth factor receptor (EGFR) and erb-b2 receptor tyrosine kinase (HER2), or anaplastic lymphoma kinase (ALK), and b) an autophagy inducer as defined above, and pharmaceutically acceptable carriers, excipients and/or vehicles.

As used herein, the term "active ingredient" refers to a component that is intended to furnish pharmacological activity or other direct effect.

The term "pharmaceutically acceptable carrier, excipients and/or vehicles" refers to a non-toxic carrier, excipient or vehicle that does not destroy the pharmacological activity of the compound with which it is formulated.

An excipient is any component, other than the active substance(s), present in a medicinal product or used in the manufacture of the product. The intended function of an excipient is to act as the carrier or as a component of the carrier of the active substance(s) and, in so doing, to contribute to product attributes such as stability, biopharmaceutical profile, appearance and patient acceptability and to the ease with which the product can be manufactured. Usually, more than one excipient is used in the formulation of a medicinal product. A vehicle is the carrier, composed of one or more excipients, for the active substance(s) in a liquid preparation. Excipients may aid the processing of the pharmaceutical product, protect, support, enhance stability, bioavailability, or patient acceptability, or enhance other attributes of the overall safety and effectiveness of the pharmaceutical product during storage or use. Pharmaceutically acceptable carriers, excipients or vehicles may include buffer substances or carriers for oral dosage forms.

The pharmaceutical combination product is particularly suitable for use in medical treatment, such as in cancer treatment. A further aspect thus relates to the pharmaceutical combination product for use in the treatment of cancer. In embodiments, the cancer is selected from the group comprising or consisting of breast cancer, lung cancer, particularly non-small cell lung cancer, bladder cancer, stomach cancer, particularly gastroesophageal cancer, and malignant pleural mesothelioma.

As outlined above, use of a combination of a) an inhibitor of tyrosine kinase EGFR, HER2 or ALK and b) an autophagy inducer, refers to use of two different active ingredients in combination.

A further aspect relates to an autophagy inducer as defined above for use in the treatment of cancer, wherein the autophagy inducer is administered in combination with an inhibitor of a tyrosine kinase selected from human epidermal growth factor receptor (EGFR) family members epidermal growth factor receptor (EGFR) and erb-b2 receptor tyrosine kinase (HER2), or anaplastic lymphoma kinase (ALK).

Another aspect relates to a use of an autophagy inducer as defined above for the manufacture of a pharmaceutical combination product for the treatment of cancer, wherein the autophagy inducer is administered in combination with an inhibitor of a tyrosine kinase selected from human epidermal growth factor receptor (EGFR) family members epidermal growth factor receptor (EGFR) and erb-b2 receptor tyrosine kinase (HER2), or anaplastic lymphoma kinase (ALK).

Another aspect relates to a method of treating cancer, the method comprising the step of administering to a subject a therapeutically effective amount of a) an inhibitor of a tyrosine kinase selected from human epidermal growth factor receptor (EGFR) family members epidermal growth factor receptor (EGFR) and erb-b2 receptor tyrosine kinase (HER2), or anaplastic lymphoma kinase (ALK), and b) an autophagy inducer as defined above. In the aspects relating to the medical treatment of a subject, a) the inhibitor of tyrosine kinase EGFR, HER2 or ALK; and (b) the autophagy inducer are administered in synergistically active dosages. The term "therapeutically effective amount" is used herein to mean an amount or dose sufficient to cause an improvement in a clinically significant condition in the subject. The subject may be a mammal, particularly a human.

In embodiments of all aspects disclosed herein, the inhibitor of the tyrosine kinase EGFR may be selected from the group comprising or consisting of gefitinib, erlotinib, afatinib, dacomitinib, osimertinib, carnertinib, cetuximab, matuzumab, panitumumab, and necitumumab. In embodiments of all aspects disclosed herein, the inhibitor of the tyrosine kinase EGFR may be selected from the group comprising or consisting of gefitinib, erlotinib, afatinib, dacomitinib and osimertinib. In other embodiments, the inhibitor of the tyrosine kinase EGFR may be selected from the group comprising or consisting of cetuximab, matuzumab, panitumumab, and necitumumab.

In embodiments of all aspects disclosed herein, the inhibitor of the tyrosine kinase HER2 may be selected from the group comprising or consisting of lapatinib, neratinib, tucatinib, pyrotinib, canertinib, afatinib, trastuzumab, pertuzumab, trastuzumab emtansine, and trastuzumab deruxtecan. In embodiments, the inhibitor of the tyrosine kinase HER2 may be selected from the group comprising or consisting of lapatinib, neratinib, tucatinib, pyrotinib, canertinib, and afatinib, preferably of lapatinib, neratinib and tucatinib. In other embodiments, the inhibitor of the tyrosine kinase HER2 may be selected from the group comprising or consisting of trastuzumab, pertuzumab, trastuzumab emtansine, and trastuzumab deruxtecan.

In embodiments of all aspects disclosed herein, the inhibitor of the tyrosine kinase ALK may be selected from the group comprising or consisting of ceritinib, crizotinib, alectinib, brigatinib and lorlatinib.

In preferred embodiments of all aspects disclosed herein, the autophagy inducer is selected from the group comprising or consisting of SW076956, SW063058, SW076956-35, SW076956-24, BRD1991 and BRD5631.

In embodiments of all aspects disclosed herein,
- the inhibitor of the tyrosine kinase EGFR is erlotinib, and the autophagy inducer is SW076956; or
- the inhibitor of the tyrosine kinase EGFR is erlotinib, and the autophagy inducer is SW063058; or
- the inhibitor of the tyrosine kinase HER2 is lapatinib, and the autophagy inducer is SW076956; or
- the inhibitor of the tyrosine kinase ALK is ceritinib, and the autophagy inducer is SW076956; or
- the inhibitor of the tyrosine kinase ALK is crizotinib, and the autophagy inducer is SW076956.

In an embodiment of all aspects disclosed herein, the cancer is selected from the group comprising or consisting of breast cancer, lung cancer, particularly non-small cell lung cancer, bladder cancer, stomach cancer, particularly gastroesophageal cancer, colorectal cancer, squamous cell cancer of the head and neck, and malignant pleural mesothelioma.

The term "lung cancer" generally refers to non-small cell lung cancer (NSCLC) and small cell lung cancer (SCLC). Stomach cancer, also called gastric cancer, can happen in any part of the stomach. However, most stomach cancers occur in the gastroesophageal junction, where the esophagus meets the stomach.

In some embodiments, the cancer is selected from lung cancer and breast cancer.

In embodiments, the cancer is selected from lung cancer, colorectal cancer, and squamous cell cancer of the head and neck. In some embodiments, the cancer is lung cancer, particularly non-small cell lung cancer. 20% of non-small cell lung cancer (NSCLC), which represents 85% of all lung cancers, have mutations in *EGFR (HER1).* In some embodiments, the cancer is colorectal cancer, particularly EGFR-expressing colorectal cancer. In some embodiments, the cancer is squamous cell cancer of the head and neck.

In some embodiments, the cancer is breast cancer, particularly ductal breast cancer. HER2 and EGFR are frequently overexpressed in breast cancer.

In some embodiments, the cancer is selected from breast cancer, gastroesophageal cancer and bladder cancer. In some embodiments, the cancer is breast cancer. In some embodiments, the cancer is gastroesophageal cancer. In some embodiments, the cancer is bladder cancer. About 20% of breast cancers have amplifications in *HER2 (ERBB2),* and this correlates with a more aggressive disease. HER2 amplifications are also found in about 10-30% gastroesophageal as well as 15% of bladder cancers.

In some embodiments, the cancer is lung cancer, including malignant pleural mesothelioma. ALK (anaplastic lymphoma kinase) is involved in multiple forms of lung cancer, including malignant pleural mesothelioma. ALK positive lung cancer represents around 15% of the total cases worldwide.

Unless otherwise defined, the technical and scientific terms used herein have the same meaning as commonly understood by one of ordinary skill in the art to which this invention belongs.

The examples which follow serve to illustrate the invention in more detail but do not constitute a limitation thereof.

The figures show:

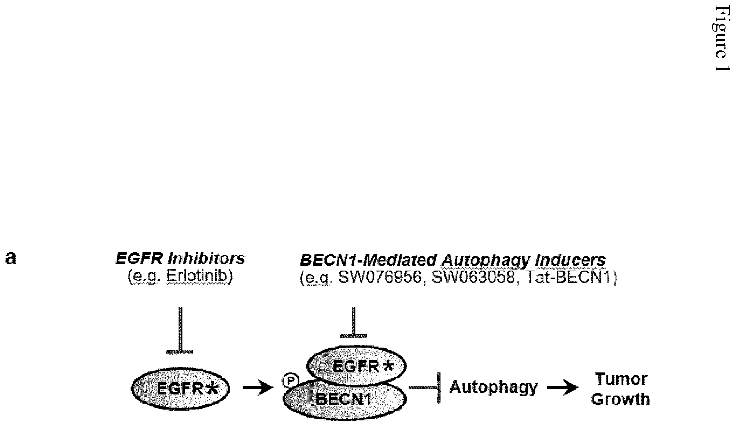

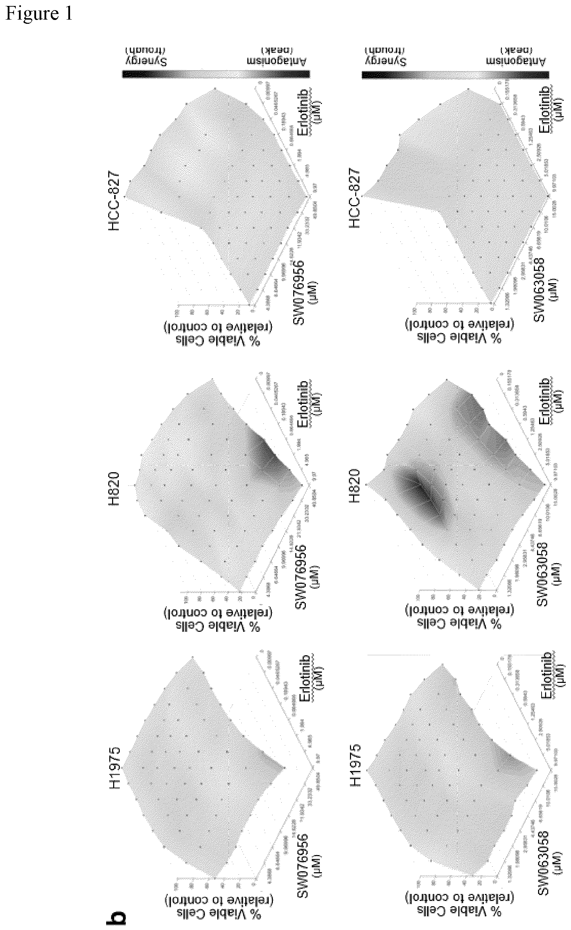

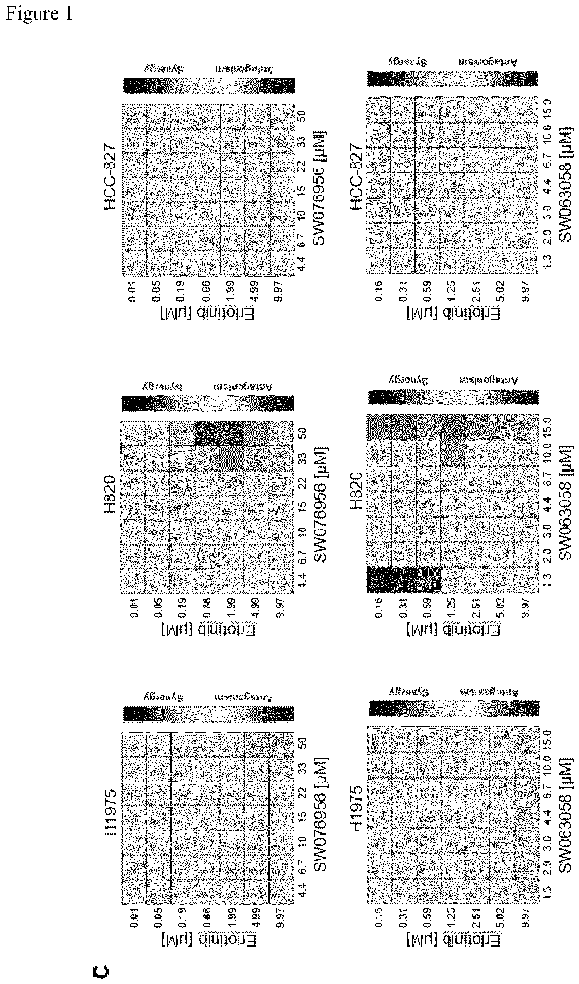

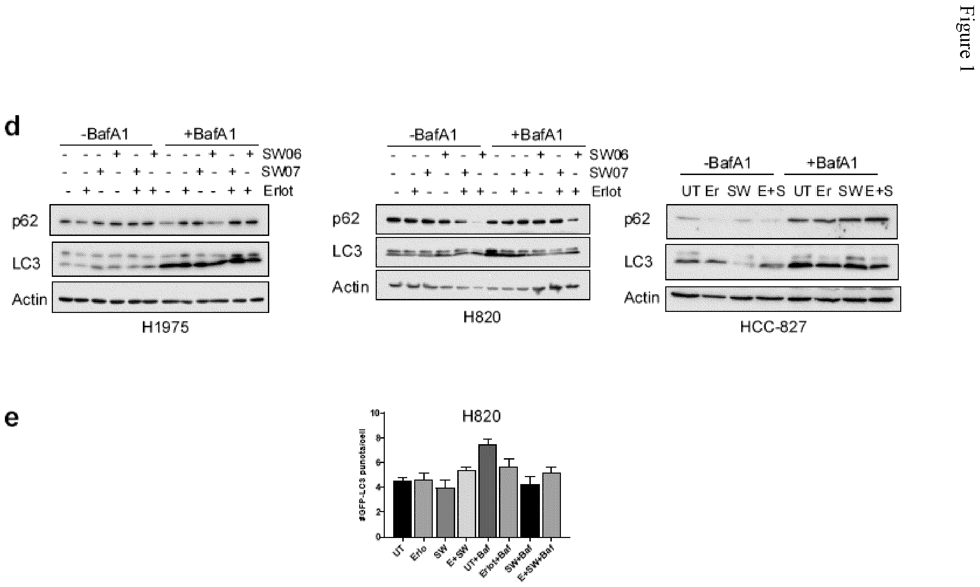

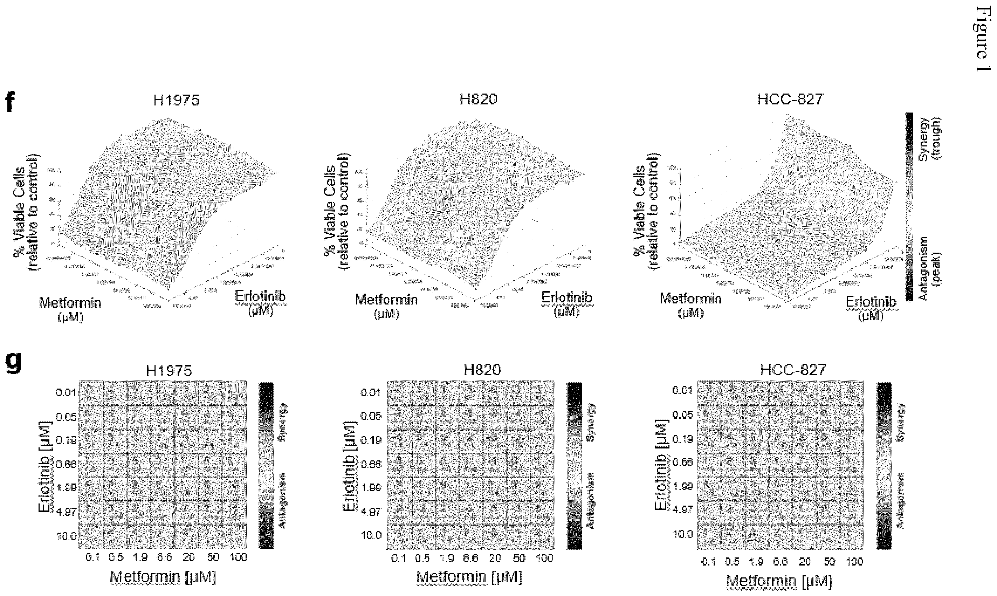

- Figure 1: in accordance with the experiments described in Example 1, illustrates the synergistic effect on the induction of autophagy and cell viability in cells treated with a combination Erlotinib, an inhibitor of the tyrosine kinase EGFR, and autophagy inducer SW076956 or SW063058 in EGFR-mutant non-small cell lung cancer cells. (a) Proposed mechanism of regulation of autophagy through the binding and phosphorylation of BECN1 by HER1/EGFR mutants and rationale for treating EGFR-mutant cancers with EGFR inhibitors in combination with autophagy inducers; (b) Combination effects of erlotinib and SW076956 treatments or of erlotinib and SW063058 treatments on cell viability of the EGFR-mutant non-small cell lung cancer cells H1975, H820 and HCC-827 were quantified 96 h after treatment of serial dilutions of single and combined compounds in three independent experiments; (c) Synergy/antagonism effects of erlotinib and SW076956 treatments or of erlotinib and SW063058 treatments were determined by a Loewe synergy model with Combenefit software of three independent experiments; (d) Combination effects of erlotinib and SW076956 treatment or of erlotinib and SW063058 treatment on autophagy induction in H1975, H820 and HCC-827 cells assessed by Western blotting; (e) number of autophagosomes assessed by numbers of GFP-LC3 puncta in H820 cells; (f) Lack of synergistic effects of erlotinib and non-BECN1-mediated autophagy inducer metformin treatment on cell viability of the EGFR-mutant non-small cell lung cancer cells H1975, H820 and HCC-827 quantified 96 h after treatment of serial dilutions of single and combined compounds in three independent experiments; (g) Effects of erlotinib and metformin treatment determined by a Loewe synergy model with Combenefit software of three independent experiments. Error bars represent the average ± SEM (*n* = 50). *, *P*<0.05; **, *P*<0.01; ***, *P*<0.001.

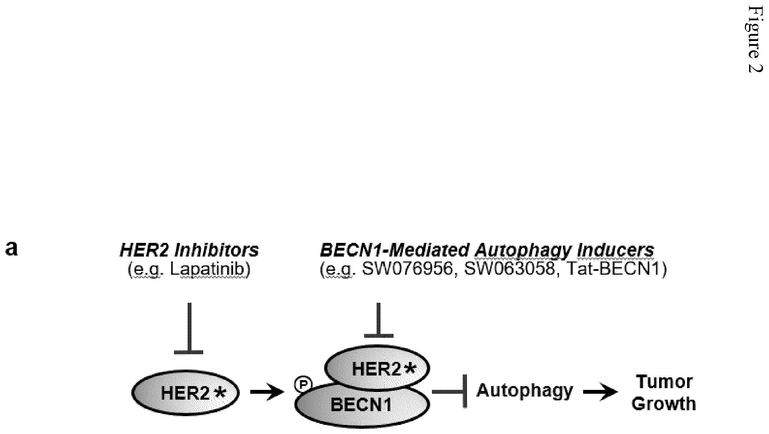

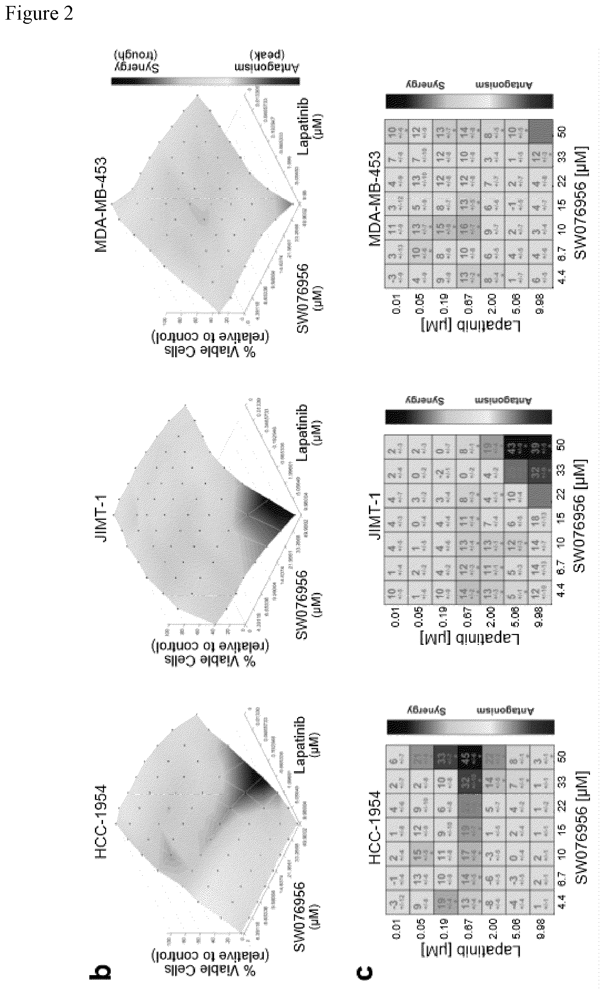

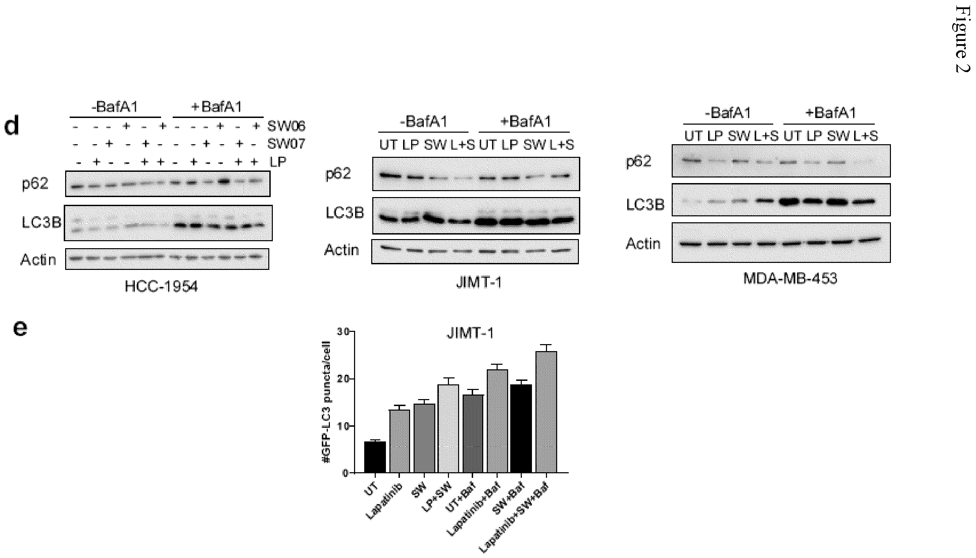

- Figure 2: in accordance with the experiments described in Example 2, illustrates the synergistic effect on the induction of autophagy and cell viability in cells treated with a combination of lapatinib, an inhibitor of the tyrosine kinase HER2, and the autophagy inducer SW076956 in HER2-deregulated breast cancer cells: (a) Proposed mechanism of regulation of autophagy through the binding and phosphorylation of BECN1 by amplified or mutant HER2 and rationale for treating deregulated HER2 cancers with HER2 inhibitors in combination with autophagy inducers; (b) Combination effects of lapatinib and SW076956 treatments on cell viability of the HER2-deregulated breast cancer cells HCC-1954, JIMT-1 and MDA-MB-453 were quantified 96 h after treatment of serial dilutions of single and combined compounds in at least three independent experiments; (c) Synergy/antagonism effects of lapatinib and SW076956 were determined by a Loewe synergy model with Combenefit software of three independent experiments in HCC-1954 and JIMT-1 and four independent experiments in MDA-MB-453 cells; (d) Combination effects of lapatinib and SW076956 treatment on autophagy induction in HCC-1954, JIMT-1 and MDA-MB-453 cells assessed by Western blotting; (e) Number of autophagosomes assessed by numbers of GFP-LC3 puncta in JIMT-1 cells. Error bars represent the average ± (*n* = 50); *, *P*<0.05; **, *P*<0.01*;* ***, *P*<0.001.

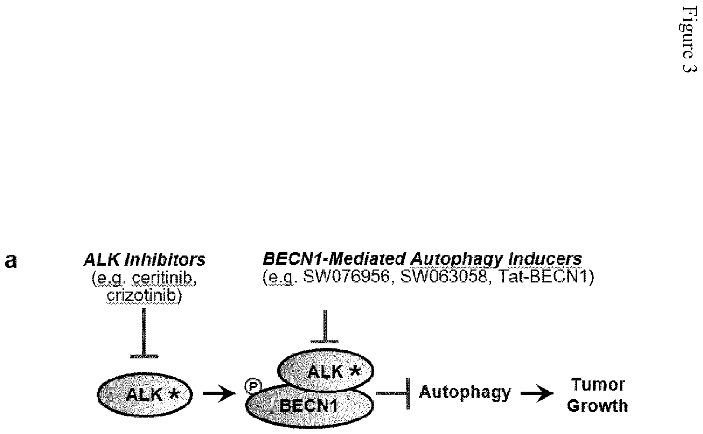

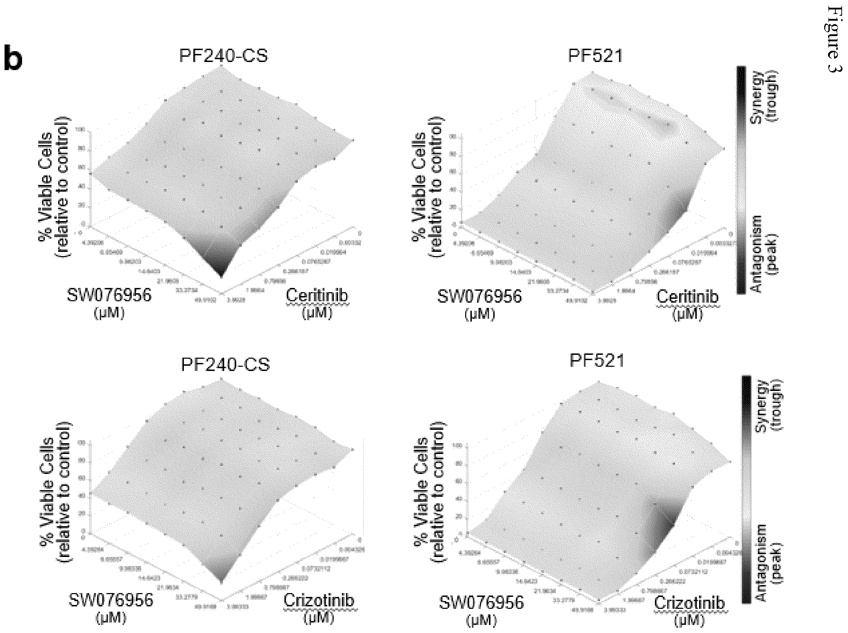

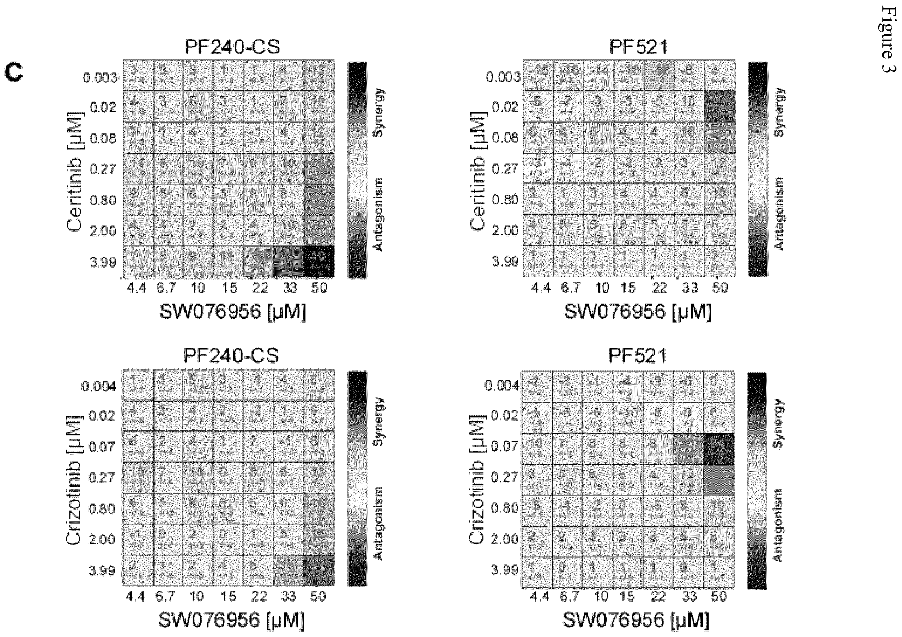

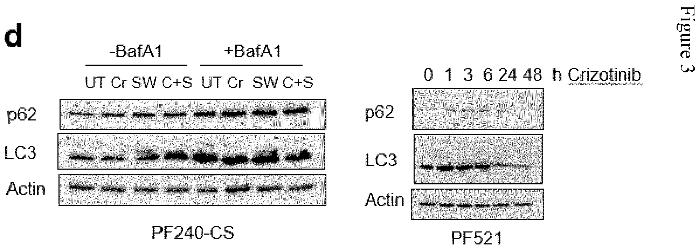

- Figure 3: in accordance with the experiments described in Example 3, illustrates the synergistic effect on the induction of autophagy and cell viability in cells treated with a combination of Ceritinib or Crizotinib, each an inhibitor of the tyrosine kinase ALK, and the autophagy inducer SW076956 in ALK-rearranged lung adenocarcinoma cells: (a) Proposed mechanism of regulation of autophagy through the binding and phosphorylation of BECN1 by rearranged anaplastic lymphoma kinase (ALK) and rationale for treating ALK-rearranged cancers with ALK inhibitors in combination with autophagy inducers; (b) Combination effects of ceritinib and SW076956 treatments or of crizoitinib and SW076956 treatments on cell viability of the ALK-rearranged lung adenocarcinoma cells PF240-CS and PF521 were quantified 96 h after treatment of serial dilutions of single and combined compounds in four independent experiments; (c) Synergy/antagonism effects of ceritinib and SW076956 or of crizotinib and SW076956 were determined by a Loewe synergy model with Combenefit software of four independent experiments; (d) Combination effects of crizotinib and SW076956 treatment on autophagy induction in PF240-CS and PF521 cells assessed by Western blotting.

### Methods and materials

### Reagents

EGFR inhibitor erlotinib was purchased from Selleckchem and dissolved at 5 mM in DMSO. HER2 inhibitor lapatinib was purchased from Biozol as a solution of 10 mM in DMSO. ALK inhibitor ceritinib was purchased from Selleckchem and dissolved at 5 mM in DMSO. ALK inhibitor crizotinib was purchased from Selleckchem and dissolved at 10 mM in DMSO. SW076956 was obtained from D&C Chemicals (Shanghai, China) and dissolved at 50 mM in DMSO. SW063068 was purchased from Merck and dissolved at 2 mM in DMSO.

### Antibodies

Antibodies were from the following sources: Santa Cruz (Actin-HRP, HRP-conjugated secondary antibodies), Novus Biologicals (LC3B), Progen (p62).

### Cell culture

EGFR-mutant non-small cell lung cancer cells H1975, H820 and HCC-827 were obtained from ATCC. HER2-deregulated breast cancer cells HCC-1954, JIMT-1 and MDA-MB-453 were obtained from ATCC through Prof. Dr. Stefan Wiemann (DKFZ). ALK-rearranged lung adenocarcinoma cells PF240-CS and PF521 were kindly provided by Dr. Balazs Hegedüs (Ruhrlandklinik, University Hospital Essen).

Cell lines were maintained in their corresponding medium supplemented with 10% (v/v) fetal bovine serum (FBS) (Gibco, 10500064) and 1% (v/v) Penicillin-Streptomycin (Gibco, 15140122) in a humidified incubator at 37 °C and 5% CO₂. The growth medium for PF240-CS, PF521 and MDA-MB-453 cells was Dulbecco's Modified Eagle's Medium (DMEM) (Gibco, 41965062), for H1975, H820, HCC-827 and HCC-1954 cells was RPMI (Gibco, 61870044) and for JIMT-1 was Advanced DMEM/F-12 (Gibco, 12634028). Cells were routinely tested for mycoplasma by PCR.

### Cell lysates and western blotting

Cells were washed in ice-cold PBS and lysed in ice-cold lysis buffer (Tris-HCl pH7.4, 50 mM, NaCl 250 mM, Igepal 0,5%) containing protease inhibitors (cOmplete Protease Inhibitor Cocktail, Roche or Thermo Fisher Scientific, 11834101) and phosphatase inhibitors (PhosSTOP Phosphatase Inhibitor Cocktail, Roche or Thermo Fisher Scientific, 11814101) for 10 minutes at 4 °C. Cell debris was depleted by centrifugation at 16,000·g for 10 min at 4 °C and protein concentration was assessed by Bradford assay. Cleared samples were transferred to a fresh tube, mixed with 4x loading buffer containing β-mercaptoethanol, boiled for 10 min at 95 °C and analyzed by Western blotting. Samples were loaded into a SDS-PAGE gel and transferred to a 0.45 µm PVDF membrane (Roth). Membranes were blocked 1 h in 5% skim dry-milk in TBST (blocking buffer) and incubated with the corresponding antibodies diluted in 3% BSA-TBST overnight at 4 °C. After washing 3 times in TBST membranes were incubated in secondary HRP-conjugated antibodies (1:5000 in blocking buffer) for 45 min at room temperature. Upon additional washing membranes were developed and imaged using FUSION software and imaging system.

### Autophagy assays

Autophagy activity was measured using the following assays: (1) Western blotting analysis of p62 levels and LC3B-I/II ratio. (2) Quantification of the number of autophagosomes by GFP-LC3 puncta formation. Cells were seeded in a 6-well plate and transfected the next day with the pBABE-GFP-LC3 plasmid. 24 h later cells were split into a black imaging 96-well plate (Eppendorf). After treatment cells were fixed in 4% PFA in PBS. Microscopic quantification was performed in the presence or absence of 100 nM Bafilomycin A1.

### Semi-automatic drug screening in cell lines

Compounds were dissolved in DMSO to create a concentration of 10 mM and were dispensed into white-bottom sterile polystyrene 384-well plates (Corning, 3570) at nine concentrations ranging from 5 nM to 50 µM in triplicate, using a Tecan D300e Digital Dispenser (Tecan, Switzerland / HP Inc., CA, USA) and D300e Control software. Negative controls with only DMSO were also included. To ensure capturing the linear growth phase of cells during drug treatment, cell number and assay period were optimized by seeding each cell line in triplicate at three different cell densities, ranging from 100 to 600 cells per well, in 30 µl of cell culture medium for 96 h. For drug screening experiments, 300 cells per well were seeded for H1975, H820 and HCC-827 cells, 500 cells/well for PF240-CS and PF521 cells and 600 cells/well for HCC-1954, JIMT-1 and MDA-MB-453 cells in 30 µl of culture medium using a Multidrop Combi dispenser (Thermo Fisher Scientific, MA, USA) and assay plates were incubated at 37 °C and 5% CO₂ for 4 days (96 h). The CellTiter-Glo assay (Promega) was performed to determine cell viability using a Promega GloMax Explorer microplate reader.

### Example 1: EGFR inhibitor erlotinib and autophagy inducer are synergistic in vitro

High-throughput drug combination screens for EGFR inhibitor erlotinib and autophagy inducers SW076956 and SW063058 were performed in EGFR-mutant non-small cell lung cancer cells H1975, H820 and HCC-827, testing 7 concentrations of each drug and their combinations, and measuring the cell viability after 96 hours of treatment. Potential synergy/antagonism was analyzed using the Loewe model in Combenefit software. As can be taken from Figure 1b and 1c, highly significant synergy effects of erlotinib and SW076956, as well as erlotinib and SW063058 were observed in H820 cells and also significant synergism in H1975 and HCC-827 cells.

Further, the effects of EGFR inhibitor erlotinib and autophagy inducer SW076956 alone or in combination on autophagy in H1975, H820 and HCC-827 cancer cells were tested. As can be taken from Figure 1d, the results confirmed that erlotinib and SW076956 induced autophagic flux as detected by the levels of p62 and lipidated LC3B by western blotting. As can be taken from Figure 1e, the results were confirmed in H820 cells by autophagosome numbers using the GFP-LC3 reporter.

Control testing of drug combination screens were performed in EGFR-mutant non-small cell lung cancer cells H1975, H820 and HCC-827 for the EGFR inhibitor erlotinib and the non-specific autophagy inducer metformin, which is known to be able to induce autophagy independently of BECN1. As can be taken from Figure 1f and 1g, no synergy of a combination of erlotinib and metformin was observed in either of H1975, H820 or HCC-827 cells.

### Example 2: HER2 inhibitor lapatinib and autophagy induction are synergistic in vitro

A high-throughput drug combination screen for lapatinib and SW076956 was performed in HER2-deregulated breast cancer cells HCC-1954, JIMT-1 and MDA-MB-453, testing 7 concentrations of each drug and their combinations, and measuring the cell viability after 96 hours of treatment. Potential synergy/antagonism was analyzed using the Loewe model in Combenefit software. As can be taken from Figure 2b and 2c, significant synergy of lapatinib and SW076956 was observed in HCC-1954 and JIMT-1 cells, and significant synergy of lapatinib and SW076956 was also observed in MDA-MB-453 cells.

Further, the effects of HER2 inhibitor lapatinib and autophagy inducer SW076956 alone or in combination on autophagy in HCC-1954, JIMT-1 and MDA-MB-453 cancer cells were tested. As can be taken from Figure 2d, the results confirmed that lapatinib and SW076956 induced autophagic flux as detected by the levels of p62 and lipidated LC3B by western blotting. As can be taken from Figure 2e, the results were confirmed in JIMT-1 cells by autophagosome numbers using the GFP-LC3 reporter.

### Example 3: ALK inhibition and autophagy induction are synergistic in vitro.

High-throughput drug combination screens for tyrosine kinase ALK inhibitor ceritinib and SW076956 and for tyrosine kinase ALK inhibitor crizotinib and SW076956 were performed in ALK-rearranged lung adenocarcinoma cells PF240-CS and PF521, testing 7 concentrations of each drug and their combinations, and measuring the cell viability after 96 hours of treatment. Potential synergy/antagonism was analyzed using the Loewe model in Combenefit software. As can be taken from Figure 3b and 3c, synergistic effects of ceritinib and SW076956 and of crizotinib and SW076956 were observed in PF240-CS cells, while in PF521 cells significant synergistic, but also some antagonistic effects were observed in PF521 cells.

Further, the effects of ALK inhibitor crizotinib and autophagy inducer SW076956 alone or in combination on autophagy in PF240-CS and PF521 cancer cells were tested. As can be taken from Figure 3d, the results confirmed that crizotinib and SW076956 induced autophagic flux as detected by the levels of p62 and lipidated LC3B by western blotting.

In summary, these results show that inhibitors of the tyrosine kinases EGFR, HER2 or ALK and BECN1-mediated autophagy inducers act synergistically in cancers. Thus, the results indicate that a combination product comprising an inhibitor of the tyrosine kinase EGFR, HER2 or ALK and an autophagy inducer, particularly a BECN1-mediated autophagy inducer, can be usable in the treatment of cancer.

## Claims

1. A pharmaceutical combination product comprising:
a) an inhibitor of a tyrosine kinase selected from
- human epidermal growth factor receptor (EGFR) family members epidermal growth factor receptor (EGFR) and erb-b2 receptor tyrosine kinase (HER2), or
- anaplastic lymphoma kinase (ALK); and
b) an autophagy inducer selected from the group comprising:
- SW076956 (N-(4-(5-(furan-2-yl)-1-(4-methylbenzoyl)-4,5-dihydro-1H-pyrazol-3-yl)phenyl)methanesulfonamide);
- SW063058 (7-Bromo-5-(4-fluorophenyl)-1,3,4,5-tetrahydro-4-(2-iodobenzoyl)-2H-1,4-benzodiazepin-2-one);
- SW076956-35 (isopropyl (4-(1-(4-bromobenzoyl)-5-phenyl-4,5-dihydro-1H-pyrazol-3-yl)phenyl)carbamate);
- SW076956-24 (tert-butyl (4-(1-(4-bromobenzoyl)-5-phenyl-4,5-dihydro-1H-pyrazol-3-yl)phenyl)carbamate);
- BRD1991 (3,5-dichloro-N-((10-(1-hydroxypropan-2-yl)-8,12-dimethyl-11-oxo-7,8,9,10,11,12-hexahydro-5H-benzo[9,10][1]oxa[5]azacycloundecino[7,8-b]indol-7-yl)methyl)-N-methylbenzamide);
- BRD5631 (N-[(3S,6S,7S)-7-methoxy-3,6,9-trimethyl-10-oxo-3,4,5,6,7,8,9,10-octahydro-2H-1,4,9-benzoxadiazacyclododecin-12-yl]-[1,1'-biphenyl]-4-carboxamide);
- Tat-Beclin-1;
- Tat-Beclin-1 peptide;
- Tat-Beclin-1-D11 peptide;
- Tat-Beclin-1-L11 peptide;
- Tat-vFLIPα2; and
- Tat-vFLIPα4,
or a pharmaceutically acceptable salt or hydrate or prodrug thereof.

2. The pharmaceutical combination product according to claim 1, wherein the inhibitor of the tyrosine kinase EGFR is selected from the group comprising:
- erlotinib (183321-74-6; *N*-(3-ethynylphenyl)-6,7-bis(2-methoxyethoxy)quinazolin-4-amine),
- gefitinib (184475-35-2; *N*-(3-chloro-4-fluorophenyl)-7-methoxy-6-(3-morpholin-4-ylpropoxy)quinazolin-4-amine),
- afatinib (BIBW2992; *(E)-N-[4-(3-chloro-4-fluoroanilino)-7-[(3S)-oxolan-3-*yl]oxyquinazolin-6-yl]-4-(dimethylamino)but-2-enamide),
- dacomitinib (1042385-75-0; (*E*)-*N*-[4-(3-chloro-4-fluoroanilino)-7-methoxyquinazolin-6-yl]-4-piperidin-1-ylbut-2-enamide) preferably in the form of monohydrate,
- osimertinib (AZD-9291; *N*-[2-[2-(dimethylamino)ethyl-methylamino]-4-methoxy-5-[[4-(1-methylindol-3-yl)pyrimidin-2-yl]amino]phenyl]prop-2-enamide), and
- canertinib, also denoted CI-1033 or*N-*[4-(3-chloro-4-fluoroanilino)-7-(3-morpholin-4-ylpropoxy)quinazolin-6-yl]prop-2-enamide,
- cetuximab,
- matuzumab,
- panitumumab,
- necitumumab,
or a pharmaceutically acceptable salt or hydrate or prodrug thereof.

3. The pharmaceutical combination product according to claim 1, wherein the inhibitor of the tyrosine kinase HER2 is selected from the group comprising:
- lapatinib (GW572016; *N*-[3-chloro-4-[(3-fluorophenyl)methoxy]phenyl]-6-[5-[(2-methylsulfonylethylamino)methyl]furan-2-yl]quinazolin-4-amine),
- neratinib (HKI-272; (*E*)-*N*-[4-[3-chloro-4-(pyridin-2-ylmethoxy)anilino]-3-cyano-7-ethoxyquinolin-6-yl]-4-(dimethylamino)but-2-enamide),
- tucatinib (ONT-380; 6-*N*-(4,4-dimethyl-5*H*-1,3-oxazol-2-yl)-4-N-[3-methyl-4-([1,2,4]triazolo[1,5-a]pyridin-7-yloxy)phenyl]quinazoline-4,6-diamine),
- pyrotinib (SHR-1258; (*E*)-N-[4-[3-chloro-4-(pyridin-2-ylmethoxy)anilino]-3-cyano-7-ethoxyquinolin-6-yl]-3-[(2R)-1-methylpyrrolidin-2-yl]prop-2-enamide),
- canertinib (CI-1033, *N*-[4-(3-chloro-4-fluoroanilino)-7-(3-morpholin-4-ylpropoxy)quinazolin-6-yl]prop-2-enamide), and
- afatinib (BIBW2992; (*E*)-*N*-[4-(3-chloro-4-fluoroanilino)-7-[(3*S*)-oxolan-3-yl]oxyquinazolin-6-yl]-4-(dimethylamino)but-2-enamide),
- trastuzumab,
- pertuzumab,
- trastuzumab emtansine,
- trastuzumab deruxtecan,
or a pharmaceutically acceptable salt or hydrate or prodrug thereof.

4. The pharmaceutical combination product according to claim 1, wherein the inhibitor of the tyrosine kinase ALK is selected from the group comprising:
- ceritinib, also denoted LDK-378 or 5-chloro-2-*N*-(5-methyl-4-piperidin-4-yl-2-propan-2-yloxyphenyl)-4-*N*-(2-propan-2-ylsulfonylphenyl)pyrimidine-2,4-diamine,
- crizotinib, also denoted PF-02341066 or 3-[(1*R*)-1-(2,6-dichloro-3-fluorophenyl)ethoxy]-5-(1-piperidin-4-ylpyrazol-4-yl)pyridin-2-amine,
- alectinib, also denoted 9-ethyl-6,6-dimethyl-8-(4-morpholinopiperidin-1-yl)-11-oxo-6,11-dihydro-5H-benzo[b]carbazole-3-carbonitrile,
- brigatinib, also denoted AP26113 or 4-amino-3-chloro-N-(4-(dimethylphosphinyl)phenyl)-*N'*-(2-methoxy-5-methylphenyl) pyrimidine-2-carboximidamide,
- lorlatinib, also denoted 3-[(2,6-difluorophenyl)methyl]-8-methyl-6-(4-morpholinopiperidin-1-yl)-2,4-dioxo-1,2,3,4-tetrahydropyrido[4,3-d]pyrimidine-7-carboxamide,
or a pharmaceutically acceptable salt or hydrate or prodrug thereof.

5. The pharmaceutical combination product according to any one of claims 1 to 4, wherein the autophagy inducer is selected from the group comprising SW076956, SW063058, SW076956-35, SW076956-24, BRD1991 and BRD563 1.

6. The pharmaceutical combination product according to any one of claims 1 to 5, wherein:
- the inhibitor of the tyrosine kinase EGFR is erlotinib, and the autophagy inducer is SW076956; or
- the inhibitor of the tyrosine kinase EGFR is erlotinib, and the autophagy inducer is SW063058; or
- the inhibitor of the tyrosine kinase HER2 is lapatinib, and the autophagy inducer is SW076956; or
- the inhibitor of the tyrosine kinase ALK is ceritinib, and the autophagy inducer is SW076956; or
- the inhibitor of the tyrosine kinase ALK is crizotinib, and the autophagy inducer is SW076956.

7. The pharmaceutical combination product according to any one of claims 1 to 6, wherein the combination product comprises the inhibitor of the tyrosine kinase and the autophagy inducer as separate entities or in a single composition.

8. The pharmaceutical combination product according to any one of claims 1 to 7, wherein the pharmaceutical combination product comprises as an active ingredient a) an inhibitor of a tyrosine kinase selected from human epidermal growth factor receptor (EGFR) family members epidermal growth factor receptor (EGFR) and erb-b2 receptor tyrosine kinase (HER2), or anaplastic lymphoma kinase (ALK), and b) an autophagy inducer as defined in claim 1, and pharmaceutically acceptable carriers, excipients and/or vehicles.

9. The pharmaceutical combination product according to any one of claims 1 to 8, for use in the treatment of cancer.

10. The pharmaceutical combination product according to claim 9, wherein the cancer is selected from the group comprising breast cancer, lung cancer, particularly non-small cell lung cancer, bladder cancer, stomach cancer, particularly gastroesophageal cancer, colorectal cancer, squamous cell cancer of the head and neck, and malignant pleural mesothelioma.

11. An autophagy inducer as defined in claim 1 for use in the treatment of cancer, wherein the autophagy inducer is administered in combination with an inhibitor of a tyrosine kinase selected from human epidermal growth factor receptor (EGFR) family members epidermal growth factor receptor (EGFR) and erb-b2 receptor tyrosine kinase (HER2), or anaplastic lymphoma kinase (ALK).

12. Use of an autophagy inducer as defined in claim 1 for the manufacture of a pharmaceutical combination product for the treatment of cancer, wherein the autophagy inducer is administered in combination with an inhibitor of a tyrosine kinase selected from human epidermal growth factor receptor (EGFR) family members epidermal growth factor receptor (EGFR) and erb-b2 receptor tyrosine kinase (HER2), or anaplastic lymphoma kinase (ALK).

13. A method of treating cancer, the method comprising the step of administering to a subject a therapeutically effective amount of a) an inhibitor of a tyrosine kinase selected from human epidermal growth factor receptor (EGFR) family members epidermal growth factor receptor (EGFR) and erb-b2 receptor tyrosine kinase (HER2), or anaplastic lymphoma kinase (ALK), and b) an autophagy inducer as defined in claim 1.

14. The autophagy inducer for use according to claim 11, the use according to claim 12, or the method according to claim 13, wherein:
- the inhibitor of the tyrosine kinase EGFR is erlotinib, and the autophagy inducer is SW076956; or
- the inhibitor of the tyrosine kinase EGFR is erlotinib, and the autophagy inducer is SW063058; or
- the inhibitor of the tyrosine kinase HER2 is lapatinib, and the autophagy inducer is SW076956; or
- the inhibitor of the tyrosine kinase ALK is ceritinib, and the autophagy inducer is SW076956; or
- the inhibitor of the tyrosine kinase ALK is crizotinib, and the autophagy inducer is SW076956.

15. The autophagy inducer for use according to claim 11, the use according to claim 12, or the method according to claim 13, wherein the cancer is selected from the group comprising breast cancer, lung cancer, particularly non-small cell lung cancer, bladder cancer, stomach cancer, particularly gastroesophageal cancer, and malignant pleural mesothelioma.
